# EUROPEAN PATENT APPLICATION

(11) **EP 4 563 080 A2**
(43) Date of publication of application: **04.06.2025**
(21) Application number: 24215326.0
(22) Date of filing: 26.11.2024
(51) Int. Cl.: A61B 5/00, A61B 5/0205, G16H 40/63, G16H 40/67, G16H 80/00

(54) **INTELLIGENT MEDICAL SYSTEM AND APPLICATION METHOD THEREOF**

(30) Priority: 30.11.2023 TW 112146691
(71) Applicant: iRobot Medicine Technology CO., LTD., Taipei City 114 (TW)
(72) Inventor: MI, Hsin-Wu, Taipei City (TW); LIAO, En-Chih, Taipei City (TW); LIN, Tsung-Han, Taipei City (TW); KUO, Zih-Cian, Taipei City (TW)
(74) Representative: Cabinet Chaillot

(57) **Abstract**

An intelligent medical system (1) comprises: an emergency department subsystem (3), a health information subsystem (4), and an integrated device (2) comprising: a touch display panel (23), a light transceiver, a temperature detector (37), a full-color LED (26), a strap (22), a flexible light-guide (25) optically coupled to the full-color LED (26), a control unit (30), a data transmission wireless network communication device, and an identification wireless network communication device. Various application methods of the intelligent medical system (1) include: pairing steps, triage steps, diagnosis and treatment steps, payment steps, steps for receiving medication, and steps for returning an integrated device (2). The present invention pairs the integrated device (2) with a patient's identity, and through real-time transmission of information such as vital signs, position, and triage identification signals to an intelligent healthcare status dashboard (15), healthcare personnel can promptly understand the patient's conditions and control all relevant processes.

## Description

The present invention relates to a medical system, in particular to an intelligent medical system and application method thereof, which utilizes an integrated device with a triage function, such as an intelligent medical bracelet. After paired, the integrated device can instantly transmit patient-related information including vital signs, positioning, triage signals, etc. to the intelligent medical system of the present invention. This can improve the adaptability of the medical system through real-time information transmission and data analysis, while providing patients with faster and more personalized medical services.

Currently, hospitals with emergency units face a huge volume of patients every day, putting the hospitals under extreme work pressure. Emergency patients have different medical conditions and varying degrees of urgency, which can change at any time. Under such circumstances, hospitals, physicians, and emergency patients must follow complex medical processes.

Therefore, there is an urgent need to eliminate the delays and tedious waiting for patients, integrate existing hospital and medical processes, and increase the level of intelligent automation in emergency departments. In this era of intelligent healthcare care, it is crucial to help hospitals, physicians and emergency patients overcome these challenges together. Therefore, how to introduce advanced automation technology, especially an intelligent process management system, is currently an urgent issue for the emergency medical system to solve, so as to help facilitate and improve the efficiency of the admission, diagnosis, treatment, and discharge processes, to help meet the evolving needs of emergency patients, to help hospitals, physicians, and emergency patients complete the processes swiftly and correctly; and to help make hospital and healthcare processes more intelligent/efficient and reduce the burden on them in the processes to provide a better healthcare experience.

### SUMMARY OF THE INVENTION

In view of the foregoing shortcomings and deficiencies of the present technology, the present invention provides an intelligent medical system, comprising:
an integrated device, comprising:
   a touch display panel positioned at a top portion of the integrated device and exposed on a surface of the top portion;
   a light transceiver, located at a bottom of the integrated device and exposed on a surface of the bottom;
   a body temperature detector located at the bottom of the integrated device and exposed on the surface of the bottom;
   a strap connected between a pair of opposite sides of the integrated device for securing the integrated device to a user's wrist;
   a flexible light-guide with light-conducting capabilities and located on an outer surface of the strap;
   at least one full-color LED positioned on at least one of the opposite sides of the integrated device and optically coupled to the flexible light-guide;
   a control unit connected to the touch display panel, the light transceiver, the body temperature detector, and the flexible light-guide; the control unit providing an identification code of the integrated device and obtaining real-time vital sign data of the user from the light transceiver and the body temperature detector;
   a data transmission wireless network communication device connected to the control unit;
   an identification wireless network communication device connected to the control unit;
an emergency department subsystem connected to the integrated device via the data transmission wireless network communication device and the identification wireless network communication device;
a health information subsystem connected to the emergency department subsystem via a network and capable of accessing medical record data.

In one embodiment, the light transceiver further comprises a positioning subsystem, the positioning subsystem utilizing a positioning wireless network, the integrated device comprising a positioning wireless network communication device of the positioning wireless network, and the positioning wireless network communication device connected to the control unit;
wherein the positioning subsystem comprises plural ultra-wideband (UWB) base stations and calculates a current location of the integrated device by detecting a signal propagation delay time of each of the plural UWB base stations receiving the same UWB signal emitted by the integrated device; the signal propagation delay time being the time required for a UWB signal from the integrated device to propagate to a UWB base station.

The present invention further provides an application method of an intelligent medical system utilizing the aforesaid intelligent medical system, and the application method comprises the following steps for pairing:
step a1: a health information subsystem reading a card number of a patient's health insurance card;
step a2: the health information subsystem transmitting the card number of the health insurance card and a medical record data corresponding to the card number of the health insurance card to an emergency department subsystem;
step a3: the emergency department subsystem receiving an identification code of the patient's integrated device and the medical record data;
step a4: the emergency department subsystem binding and pairing the integrated device to the health insurance card based on the card number of the health insurance card and the identification code of the integrated device.

The present invention further provides an application method of an intelligent medical system utilizing the aforesaid intelligent medical system, and the application method comprises the following steps for triage:
step b1: an emergency department subsystem reading an identification code of a patient's integrated device and real-time vital sign data of the integrated device;
step b2: the emergency department subsystem reading, via a health information subsystem, a medical record data corresponding to the identification code of the integrated device;
step b3: the emergency department subsystem presenting and displaying the medical record data and the real-time vital sign data of the integrated device to a display device at a medical end;
step b4: the emergency department subsystem instructing medical personnel at the medical end to quickly assess, select, and determine the patient's triage via an interface of the emergency department subsystem based on the medical record data and the real-time vital sign data;
step b5: the emergency department subsystem setting the light color of the integrated device for the patient based on the patient's triage.

Attached to a patient, the paired integrated device is equivalent to his/her health insurance card for the intelligent healthcare system of the present invention, especially for the emergency department subsystem, and by using the identification wireless network communication device and the positioning subsystem, the patient's relevant real-time information can be easily imported into the hospital's existing system and medical processes. Accordingly, the patient's real-time vital sign information and the hospital's relevant medical processes can be integrated, introducing greater efficiency and intelligence into the overall healthcare system as well as effectively reducing the probability of errors made by healthcare personnel.

The integrated device can authorize the retrieval of medical records from the medical subsystem, and assist medical personnel to promptly determine the patient's triage. Based on the triage determined, the emergency department subsystem can automatically instruct to set light colors for both the multiple full-color LEDs of the integrated device and the soft light-guide, facilitating the medical personnel to know the patient's condition by direct visualization, saving time and effort and avoiding misjudgment.

The integrated device can inform the patient about the time/place of relevant medical procedures, and assist in the processes of payment, medication collection, and discharge. This integrated approach, with the integrated device as the core, enhances the automation of the emergency department by not only providing real-time data, but also analyzing a large amount of digital information through an intelligent data processing system to provide real-time reference for medical personnel; even with limited manpower, the present invention can quickly and optimally serve for a large number of patients to efficiently complete various hospital processes and effectively reduce the workload of the relevant units and personnel, while ensuring fast and correct medical processes, thereby achieving the purpose of the present invention.

### In the drawings:

FIG. 1 illustrates a functional block diagram of the intelligent medical system of the present invention;
FIG. 2 illustrates a schematic diagram of the front structure of the integrated device of the present invention;
FIG. 3 illustrates a functional block diagram of the integrated device of the present invention;
FIG. 4 illustrates a flow diagram of the pairing of the integrated device and the health insurance card of the present invention;
FIG. 5 illustrates a flowchart of the present invention for triage;
FIG. 6 illustrates a flowchart of the diagnosis and treatment of the present invention;
FIG. 7 illustrates a flow chart of the present invention for discharge payment;
FIG. 8 illustrates a discharge medication flowchart of the present invention;
FIG. 9 illustrates the discharge return process of the present invention; and
FIG. 10 illustrates the intelligent voice process of the present invention.

The technical contents, features and effects of the present invention will be clearly elucidated in the following detailed description of the preferred embodiment, accompanied by reference to the drawings.

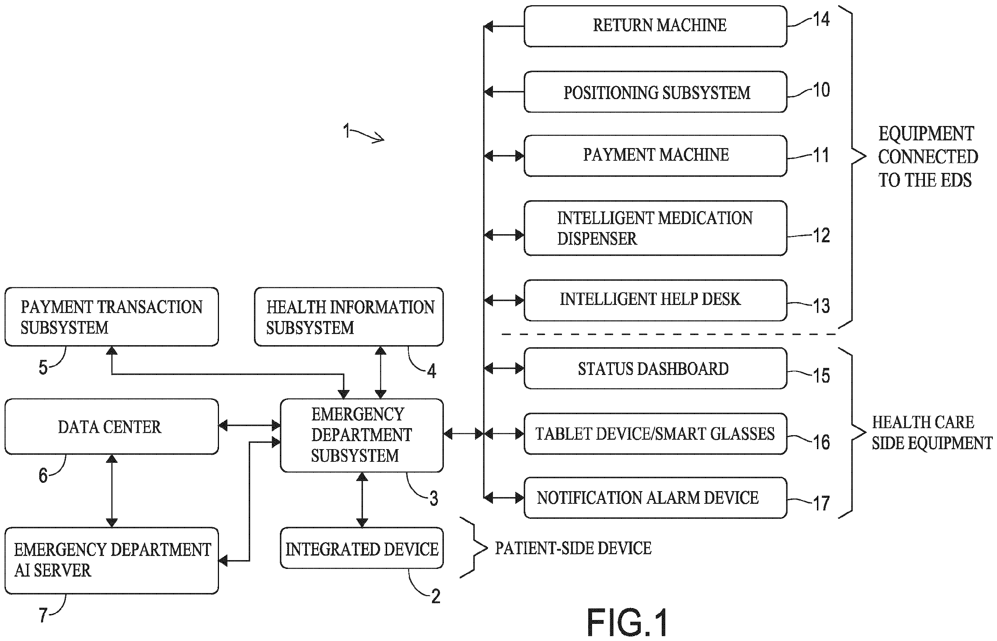

FIG. 1 illustrates a block diagram of an intelligent medical system 1 of the present invention, comprising: an integration device 2 with wireless communication capabilities, an emergency department subsystem (EDS) 3 connected to the integration device 2 via a wireless network, a health information subsystem (HIS) 4 connected to the emergency department subsystem 3 via a network, a payment transaction subsystem 5 connected to the emergency department subsystem 3 via the network, and a data center 6 connected to the emergency department subsystem 3 via the network.

The emergency department subsystem 3, being the main server of the intelligent medical system 1 of the present invention, comprises plural databases and plural servers such as: the emergency department subsystem 3 database, an application server, an in-memory database, and an emergency department AI server 7. Based on authorization of a patient's health insurance card, the Health Information Subsystem (HIS)4 allows hospital staff and medical personnel to access the patient's basic information and medical history. The payment transaction subsystem 5 is a hospital cashier system that calculates a patient's medical expenses and determines/records the medical expenses paid by the patient; the data center 6 is for storing/recording the data generated by the intelligent medical system 1 of the present invention for use by the emergency department AI server 7; the emergency department AI server 7 is mainly for providing artificial intelligence applications in conjunction with the emergency department subsystem 3 and processing the related big data, i.e., providing the related artificial intelligence response to the emergency department subsystem 3 according to its demand. For example, when the patient calls the emergency department subsystem 3 via the integration device 2 "Out of intravenous drip!" to the emergency department AI server 7 for speech recognition, and if interpreted as "the patient requests help for the intravenous drip" by speech recognition, the emergency department subsystem 3 can perform subsequent processing based on the request, for example, assigning an intelligent clinical cart or a nurse to the patient to finish treatment or replacement of the intravenous drip medication. The remainder of FIG. 1 will be described later.

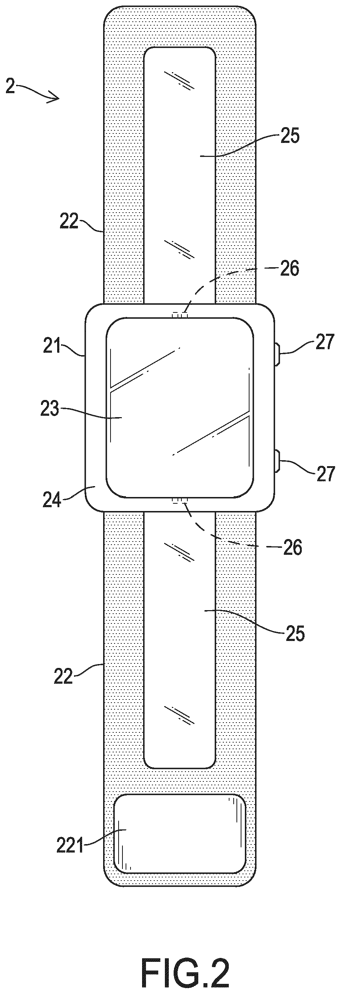

Referring to FIG. 2, FIG. 2 illustrates a schematic front view of the integrated device 2 of the present invention, which is a patient-side device worn by the patient, such as a smart medical bracelet worn on the wrist. When an identification code of the integrated device 2 is paired with a health insurance card number of the patient via the emergency department subsystem 3, the integrated device 2 is equivalent to the health insurance card for the intelligent medical system 1 of the present invention and, in particular, for the emergency department subsystem 3, facilitating the health information subsystem (HIS) 4 to be authorized by the patient's integrated device 2 to provide the medical personnel with the patient's basic information and medical history. The remainder of FIG. 2 will be described later.

As shown in FIG. 1, the intelligent medical system 1 of the present invention also includes equipment connected to the emergency department subsystem 3 via a network, including a positioning subsystem 10, a payment machine 11, an intelligent medication dispenser 12, an intelligent help desk 13, and a return machine 14, with the positioning subsystem 10 having plural wireless base stations, such as plural ultrawideband (UWB) base stations, all of which can receive the same UWB signal from the integrated device 2, and the location of the integrated device 2 is calculated based on the signal delay time related to the distance between each of the plural ultra-wideband (UWB) base stations and the integrated device 2. For example, in an implementation example, when six UWB base stations are provided in 1800 square meters, the positioning accuracy can reach 10 to 30 cm. The payment machine 11 can read an identification code of the integrated device 2 of a patient, and after the patient pays the bill, the payment machine 11 notifies the payment transaction subsystem 5 via the emergency department subsystem 3 of this transaction. The intelligent medication dispenser 12 reads an identification code of a patient's integrated device 2 and thereafter provides a prescribed medication for the patient to pick up.

The intelligent help desk 13 may receive general inquiries, including text/voice inquiries and responds to the inquiries it receives in cooperation with the emergency department subsystem 3, including text/voice responses and treatments from the medical personnel. The return machine 14 can read an identification code of a patient's integration device 2, and after the reading, the return machine 14 cooperates with the emergency department subsystem 3 to break the pairing between the identification code of the integration device 2 and the patient's health insurance card number.

The intelligent medical system 1 of the present invention also includes the health care side equipment connected to the emergency department subsystem 3 via a network, including: a status dashboard 15, a tablet device/smart glasses 16, and a notification alarm device 17, wherein the status dashboard 15 mainly receives the patient's current medical information and location information provided by the emergency department subsystem 3, and displays them on a display, allowing the medical personnel to know the patient's vital signs in real time and the treatment that has been or will be given, such as whether the patient's current condition is stable, when the patient should take the injection and medication, or when the patient should go to the X-ray, etc. The tablet device/smart glasses 16 is a portable display/communication device for medical personnel to stay in contact with the emergency department subsystem 3 at all times. When deemed necessary to alert the medical personnel, the emergency department subsystem 3 activates the notification alarm device 17 to emit light or sound.

As shown in FIG. 2, the integrated device 2 comprises a main body 21, a strap 22 connected between a pair of opposite sides of the main body 21 of the integrated device 2, a touch display panel 23 positioned on a top portion of the main body 21 of the integrated device 2, and the touch display panel 23 exposed on the surface of the top portion, a case 24, a flexible light-guide 25 with light-guiding capabilities and positioned on an outer surface of the strap 22, plural full-color light-emitting diodes (hereinafter "full-color LEDs") 26 which are positioned on the pair of opposite sides of the main body 21 of the integrated device 2 and optically coupled with the flexible light-guide 25, a button 27, and a hook-and-loop fastener 221. Alight transceiver (not shown in the drawings) is provided on a bottom surface of the integrated device 2 and exposed on the bottom surface, and a body temperature detector 37 (not shown in FIG. 2) is provided on the bottom surface of the main body 21 of the integrated device 2 and exposed on the bottom surface. The hook-and-loop fastener 221 is used to secure the strap 22 to a patient's wrist; the flexible light-guide 25 is used to extend the light emitted from the plural full-color LEDs 26 to the outer surface of the strap 22, thereby more vividly and softly displaying the light emitted from the plural full-color LEDs 26 and making it easier for people around the patient to assess the severity of the condition of the patient wearing the integrated device 2 based on the light color displayed by the flexible light-guide 25; for example, red light represents the most severe condition, followed by orange light, followed by blue light, followed by green light, with green light representing the mildest condition. The touch display panel 23 may be a thin-film transistor (TFT) liquid crystal touch panel, a quantum dot (QD) touch panel, or an active organic light-emitting diode (AMOLED) touch panel, and the touch display panel 23 may display information, enter data, or clip options. The flexible light-guide 25 may be made of flexible, translucent silicone or transparent rubber.

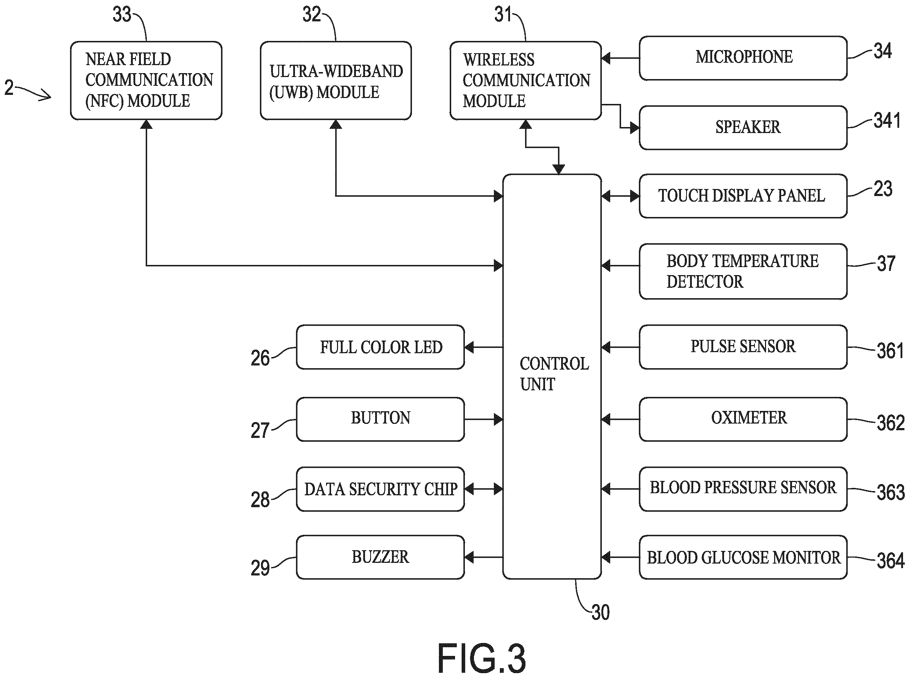

Referring to FIG. 3, FIG. 3 illustrates a functional block diagram of the integrated device 2 of the present invention, wherein the integrated device 2 further comprises a control unit 30, a wireless communication module 31 connected to the control unit 30, an ultra-wideband (UWB) module 32, a near-field communication (NFC) module 33, the touch display panel 23, plural full-color LEDs 26, the button 27, a data security chip 28, a buzzer 29, a pulse sensor 361, an oximeter 362, a blood pressure sensor 363, a blood glucose monitor 364, the body temperature detector 37, and the light transceiver (not shown), with the wireless communication module 31 also connected to a microphone 34 and a speaker 341. In one embodiment, the wireless communication module 31 may be a Wi-Fi module, a Bluetooth module, or both.

Wherein the control unit 30 may be a microcontroller (MCU), the control unit 30 may read an identification code of the integrated device 2 from its built-in memory or from other memories (not shown in the drawings) of the integrated device 2, and provide the identification code of the integrated device 2 to the NFC module 33, thereby transmitting the identification code of the integrated device to the exterior environment via the NFC module 33.

The light transceiver includes a light emitting diode (LED) and a light sensor (not shown in the drawings), the light transceiver shines light from the LED onto the skin at the user's wrist, and the light sensor senses changes in light intensity of the light penetrating through the skin and then reflecting back to the light sensor. The pulse sensor 361, the oximeter 362, the blood pressure sensor 363, and the blood glucose monitor 364 share the changes in light intensity detected by the light sensor of the light transceiver, and accordingly calculate the vital signs such as heartbeat, blood oxygen, blood pressure, and blood glucose level, etc., of the patient using the integrated device 2. In various embodiments, the pulse sensor 361, the oximeter 362, the blood pressure sensor 363, and the blood glucose monitor 364 may be a hardware chip circuit (IC) or a software program executed by the control unit 30. The body temperature detector 37, which may be an infrared temperature detector or another type of temperature detector, is utilized to detect the skin temperature on the user's wrist to check the patient's body temperature.

The buzzer 29 is utilized to alert the patient using the integrated device 2; for example, when the touch display panel 23 receives a new message or when the user is required to respond to a message, the buzzer 29 can emit a sound to alert the patient. The wireless communication module 31 is utilized for data transmission back to the emergency department subsystem 3, for example, the vital signs such as heartbeat, oxygen level, blood pressure, glucose level, and temperature of the patient measured by the pulse sensor 361, the oximeter 362, the blood pressure sensor 363, and the blood glucose monitor 364, and the body temperature detector 37 to monitor the above vital signs of the patient immediately. The wireless communication module 31 is also utilized to monitor the above vital signs of the patient.

The wireless communication module 31 may also receive messages from external sources for reference by the patient using the integrated device 2, and the output data of the wireless communication module 31 may be encrypted and the input data may be decrypted by the data security chip 28. As mentioned above, the wireless communication module 31 is connected to the microphone 34 and the speaker 341 and hence can also communicate with a matching external wireless communication module for voice communication; therefore, the wireless communication module 31 is clearly a wireless network communication device for data transmission. The NFC module 33 can be used for contactless identification at a short distance (e.g., a distance of 2 to 5 centimeters); therefore, the NFC module 33 is clearly an identification wireless network communication device. Similarly, according to the above description of the positioning subsystem 10, the ultra-wideband (UWB) module 32 is obviously a positioning wireless network communication device.

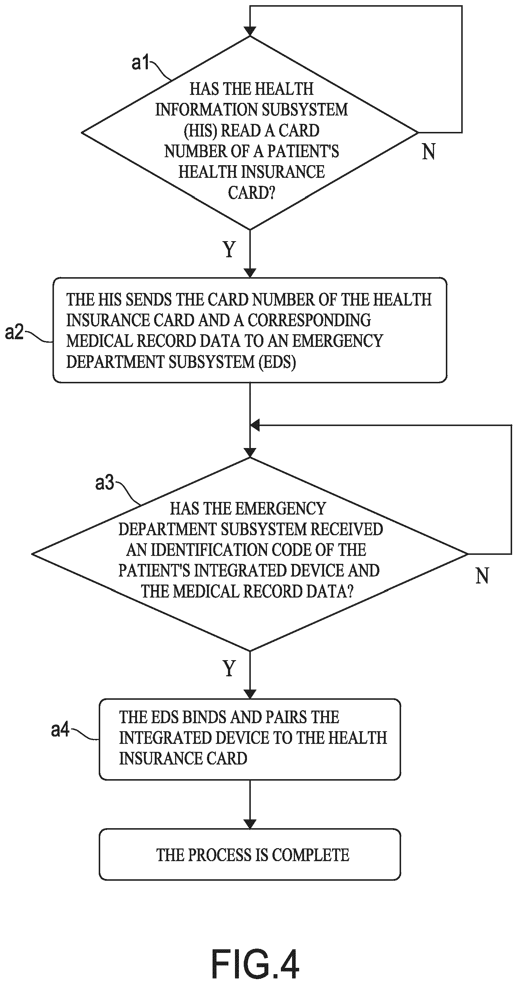

Referring to FIG. 4, FIG. 4 illustrates a flowchart of the process of pairing the integrated device 2 of the present invention with a health insurance card, comprising the following steps:
Step a1: The health information subsystem (HIS) determines whether the card number of a patient's health insurance card has been read, if not, returning to step a1;
Step a2: The HIS sends the patient's medical record data corresponding to the health insurance card number to the emergency department subsystem (EDS);
Step a3: The emergency department subsystem determines whether it has received the ID of the integrated device and the medical record data, if not, returning to step a3;
Step a4: The emergency department subsystem binds and matches the integrated device to the health insurance card according to the card number of the health insurance card and the identification code of the integrated device.

The process is hereby completed.

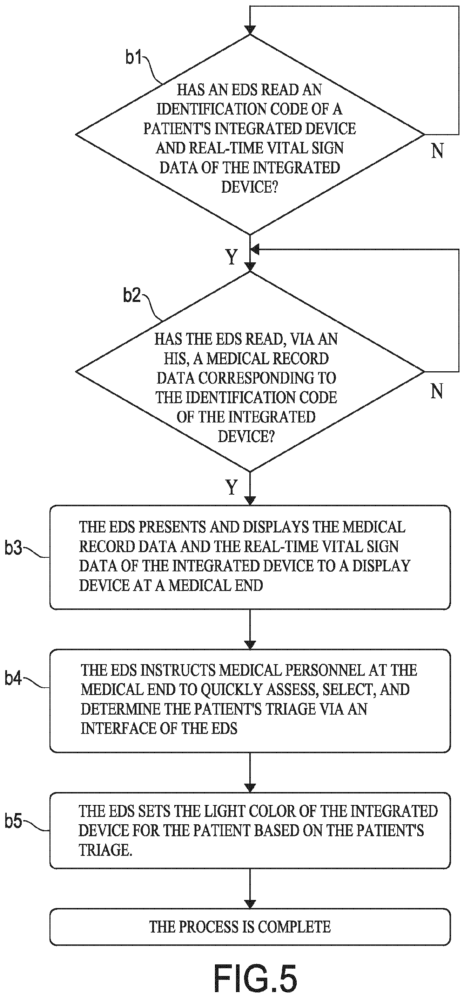

Referring to FIG. 5, FIG. 5 illustrates a flowchart of the present invention for triage with the following steps:
Step b1: The emergency department subsystem determines whether the identification code of a patient's integrated device and the real-time vital sign data of the integrated device have been read, if not, returning to step b1;
Step b2: The emergency department subsystem determines whether it has read a patient's medical record corresponding to the integrated device's identification code read by the healthcare information subsystem (HIS), if not, returning to step b2;
Step b3: The emergency department subsystem presents and displays the medical record data and the real-time vital sign data of the integrated device on a display device at a medical end;
Step b4: the emergency department subsystem instructs medical personnel at the medical end to quickly assess, select, and determine the patient's triage via an interface of the emergency department subsystem based on the medical record data and the real-time vital sign data;
Step b5: The emergency department subsystem sets the light color of the integrated device's strap for the patient based on the patient's triage.

The process is hereby completed.

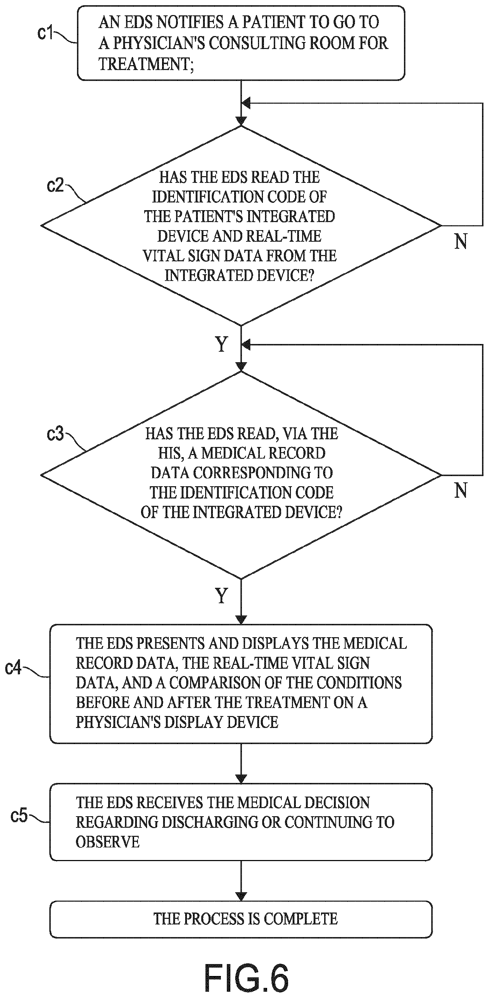

Referring to FIG. 6, FIG. 6 illustrates the diagnosis and treatment flowchart of the present invention with the following steps:
Step c1: The emergency department subsystem notifies a patient to go to a physician's consulting room for treatment;
Step c2: The emergency department subsystem determines whether the identification code of the patient's integrated device and the real-time vital sign data from the integrated device have been read, if not, returning to step c2;
Step c3: The emergency department subsystem determines whether the patient's medical record has been read by the healthcare information subsystem (HIS), if not, returning to step c3;
Step c4: The emergency department subsystem presents and displays the patient's medical record, real-time vital sign data from the integrated device, and a comparison of the patient's conditions before and after treatment on a physician's display;
Step c5: The emergency department subsystem receives the physician's input on whether to discharge the patient or continue to observe;

The process is hereby completed.

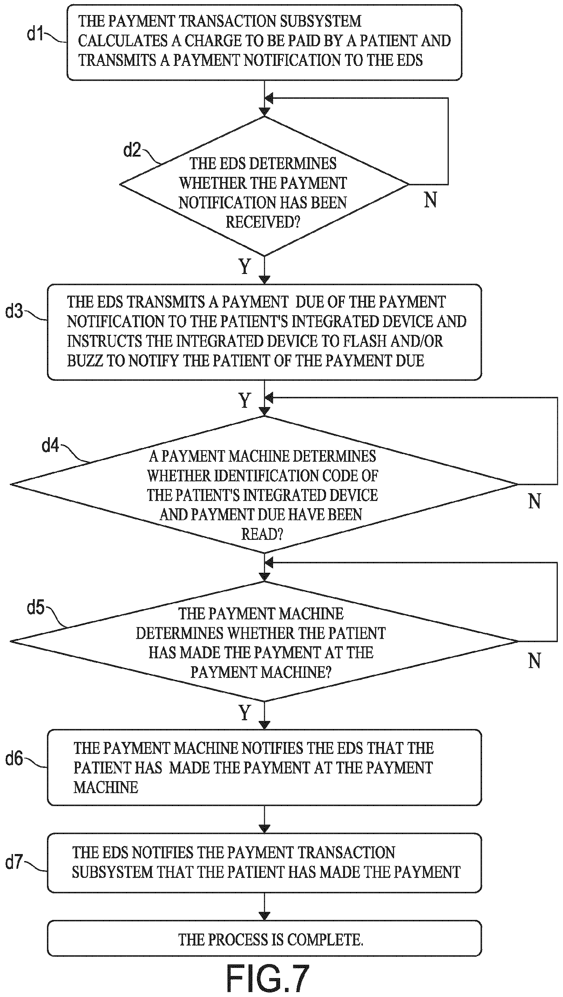

Referring to FIG. 7, FIG. 7 illustrates the discharge payment process of the present invention with the following steps:
Step d1: The payment transaction subsystem calculates a charge to be paid by a patient and transmits a payment notification to the emergency department subsystem;
Step d2: the emergency department subsystem acknowledges whether the payment notification has been received, if not, returning to step d2;
Step d3: the emergency department subsystem transmits a payment due of the payment notification to the patient's integrated device and instructs a full-color LED of the integrated device to flash and/or instructs a buzzer of the integrated device to sound to notify the patient of the payment due;
Step d4: A payment machine determines whether identification code of the patient's integrated device and payment due have been read, if not, returning to step d4;
Step d5: The payment machine determines whether the patient has made the payment at the payment machine, if not, returning to step d5;
Step d6: The payment machine notifies the emergency department subsystem that the patient has made the payment at the payment machine;
Step d7: The emergency department subsystem notifies the payment transaction subsystem that the patient has made the payment;

The process is hereby completed.

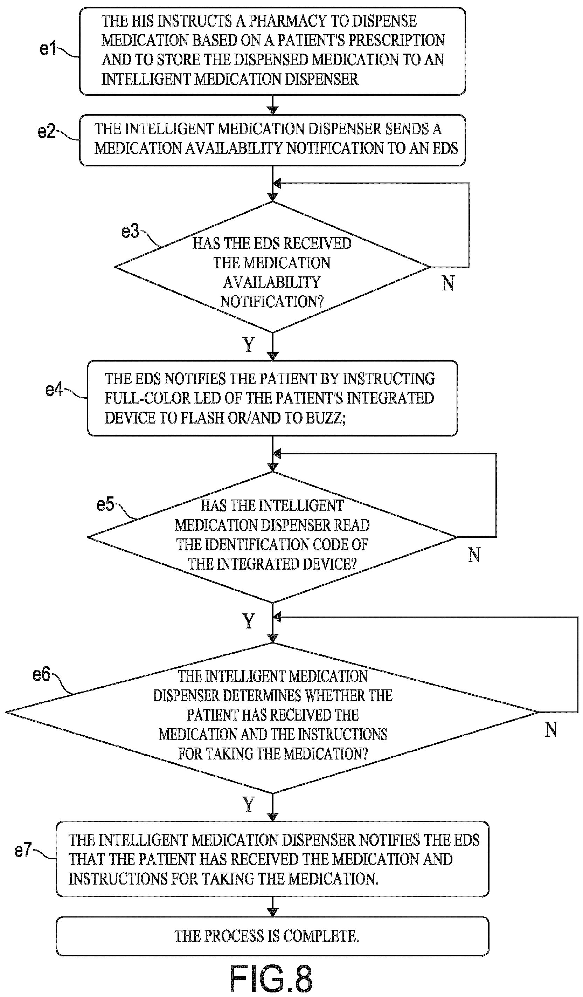

Please refer to FIG. 8, which illustrates the discharge medication receiving process of the present invention with the following steps:
Step e1: The health information subsystem (HIS) instructs a pharmacy to dispense medication based on a patient's prescription and to store the dispensed medication to an intelligent medication dispenser;
Step e2: The HIS sends a medication availability notification to the emergency department subsystem;
Step e3: The emergency department subsystem determines whether it has received the medication availability notification, if not, returning to step e3;
Step e4: The emergency department subsystem notifies the patient to receive the medication by instructing a full-color LED of the patient's integrated device to flash and/or instructing a buzzer on the patient's integrated device to sound;
Step e5: The intelligent medication dispenser determines whether the identification code of the integrated device has been read, if not, returning to step e5;
Step e6: The intelligent medication dispenser determines whether the patient has received the medication and instructions for taking the medication, if not, returning to step e6;
Step e7: The intelligent medication dispenser notifies the emergency department subsystem that the patient has received the medication and the instructions for taking the medication;

This process is hereby completed.

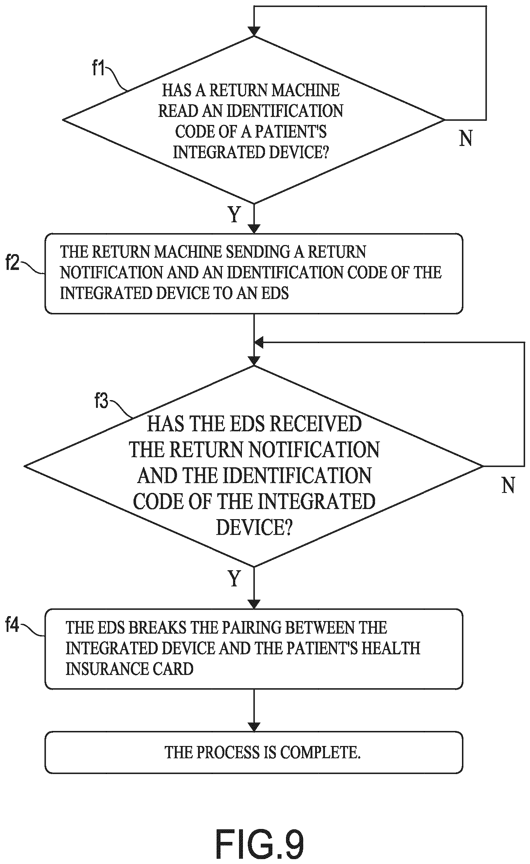

Referring to FIG. 9, FIG. 9 illustrates the discharge return process of the present invention with the following steps:
Step f1: A return machine determines whether the identification code of a patient's integrated device has been read, if not, returning to step f1;
Step f2: The return machine transmits a return notification and the identification code of the integrated device to the emergency department subsystem;
Step f3: The emergency department subsystem determines whether the return notification and the integrated device identification code have been received, if not, returning to step f3;
Step f4: The emergency department subsystem breaks the pairing between the integrated device and the patient's health insurance card;

The process is hereby completed.

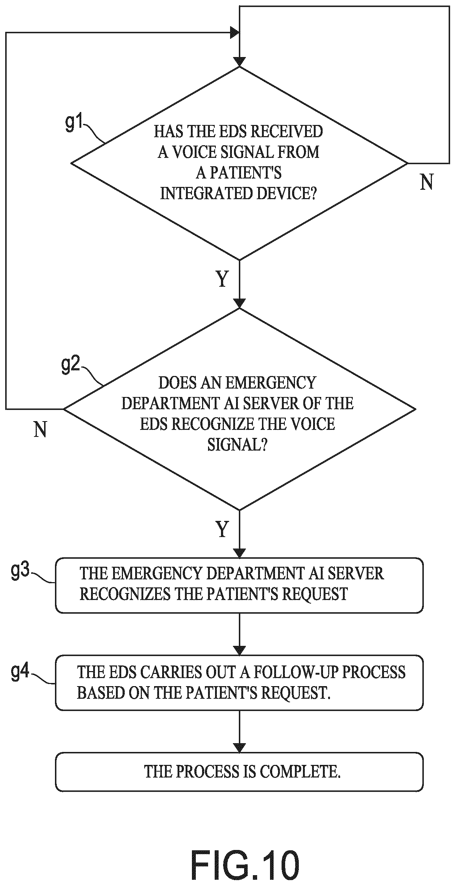

Referring to FIG. 10, FIG. 10 illustrates a flowchart of the intelligent voice flow between the integrated device and the emergency department subsystem of the present invention, including the following steps:
Step g1: The emergency department subsystem determines whether it has received a voice signal from a patient's integrated device, if not, returning to step g1;
Step g2: The emergency department AI server of the emergency department subsystem performs artificial intelligence speech recognition on the voice signal, and if unrecognizable, returning to step g1;
Step g3: The emergency department AI server of the emergency department subsystem recognizes the patient's request;
Step g4: The subsystem of the emergency department carries out follow-up process according to the patient's request;

The process is hereby completed.

The foregoing is only a better embodiment of the present invention and is not a formal limitation of the present invention. Although the present invention has been disclosed in a better embodiment as described above, it is not intended to limit the present invention, and any person skilled in the art may, without departing from the scope of the technical solutions of the present invention, use the technical content of the foregoing disclosure to make some changes or modifications to the equivalent variations. However, any simple changes, equivalent changes and modifications made to the above embodiments in accordance with the technical content of the present invention, without departing from the technical content of the present invention, still fall within the scope of the present invention.

## Claims

1. An intelligent medical system, **characterized in that** comprises:
an integrated device, comprising:
a touch display panel (23) positioned at a top portion of the integrated device (2) and exposed on a surface of the top portion;
a light transceiver, located at a bottom of the integrated device (2) and exposed on a surface of the bottom;
a body temperature detector (37) located at the bottom of the integrated device (2) and exposed on the surface of the bottom;
a strap (22) connected between a pair of opposite sides of the integrated device (2) for securing the integrated device (2) to a user's wrist;
a flexible light-guide (25) with light-conducting capabilities and positioned on an outer surface of the strap (22);
at least one full-color light-emitting diode (26) positioned on at least one of opposite sides of the integrated device (2) and optically coupled to the flexible light-guide (25);
a control unit (30) connected to the touch display panel (23), the light transceiver, the body temperature detector (37), and the flexible light-guide (25); the control unit (30) providing an identification code of the integrated device (2) and obtaining real-time vital sign data of the user from the light transceiver and the body temperature detector (37);
a data transmission wireless network communication device connected to the control unit (30);
an identification wireless network communication device connected to the control unit (30);
an emergency department subsystem (3) connected to the integrated device (2) via the data transmission wireless network communication device and the identification wireless network communication device;
a health information subsystem (4) connected to the emergency department subsystem (3) via a network and capable of accessing medical record data.

2. The intelligent medical system as claimed in claim 1, wherein
the light transceiver further comprises a light emitting diode and a light sensor, wherein light from the light emitting diode shines on skin at the user's wrist and the light sensor detects changes in light intensity of the light that penetrates into the skin and then reflects back to the light sensor;
the integrated device (2) further comprises at least one vital sign detector electrically connected to the control unit (30) and the light transceiver, and each of the at least one vital sign detector respectively calculates a vital sign value based on changes in light intensity of the light that penetrates into the skin, reflects back to the light transceiver, and is detected by the light sensor.

3. The intelligent medical system as claimed in claim 1, wherein the light transceiver further comprises a light emitting diode and a light sensor, wherein light from the light emitting diode shines on skin at the user's wrist and the light sensor detects changes in light intensity of the light that penetrates into the skin and then reflects back to the light sensor;
the control unit (30) is electrically connected to the light transceiver and executes at least one vital sign detection program, each of the at least one vital sign detection program respectively calculating a vital sign value based on the changes in light intensity of the light that penetrates into the skin, reflects back to the light transceiver, and is detected by the light sensor.

4. The intelligent medical system as claimed in any one of claims 1 to 3, wherein the vital sign data includes data on the user's body temperature, heart rate, blood oxygen level, blood pressure, or blood glucose level.

5. The intelligent medical system as claimed in claim 1, wherein
the integrated device (2) further comprises a buzzer (29) and a button (27), the buzzer (29) and the button (27) electrically connected to the control unit (30), with the button (27) located on one side of the integrated device (2) and exposed on the surface of the integrated device (2).

6. The intelligent medical system as claimed in claim 1, further comprising a positioning subsystem (10), the positioning subsystem (10) utilizing a positioning wireless network, and the integrated device (2) comprising a positioning wireless network communication device of the positioning wireless network, and the positioning wireless network communication device connected to the control unit (30);
wherein the positioning subsystem (10) comprises plural ultra-wideband (UWB) base stations and calculates a current location of the integrated device (2) by detecting a signal propagation delay time of each of the plural UWB base stations receiving the same UWB signal emitted by the integrated device (2); the signal propagation delay time being the time required for a UWB signal from the integrated device (2) to propagate to a UWB base station.

7. The intelligent medical system as claimed in claim 1, wherein
the emergency department subsystem (3) further comprises an emergency department AI server (7) with speech recognition and language comprehension capabilities, or big data processing capabilities.

8. An application method of an intelligent medical system, the application method being an application of the intelligent medical system (1) as claimed in claim 1 and comprising the following steps for pairing:
step a1: a health information subsystem (4) reading a card number of a patient's health insurance card;
step a2: the health information subsystem (4) transmitting the card number of the health insurance card and a medical record data corresponding to the card number of the health insurance card to an emergency department subsystem (3);
step a3: the emergency department subsystem (3) receiving an identification code of the patient's integrated device (2) and the medical record data;
step a4: the emergency department subsystem (3) binding and pairing the integrated device (2) to the health insurance card based on the card number of the health insurance card and the identification code of the integrated device (2).

9. An application method of an intelligent medical system, the application method being an application of the intelligent medical system (1) as claimed in claim 1 and comprising the following steps for triage:
step b1: an emergency department subsystem (3) reading an identification code of a patient's integrated device (2) and real-time vital sign data of the integrated device (2);
step b2: the emergency department subsystem (3) reading, via a health information subsystem (4), a medical record data corresponding to the identification code of the integrated device (2);
step b3: the emergency department subsystem (3) presenting and displaying the medical record data and the real-time vital sign data of the integrated device (2) to a display device at a medical end;
step b4: the emergency department subsystem (3) instructing medical personnel at the medical end to quickly assess, select, and determine the patient's triage via an interface of the emergency department subsystem (3) based on the medical record data and the real-time vital sign data;
step b5: the emergency department subsystem (3) setting the light color of the integrated device (2) for the patient based on the patient's triage.

10. An application method of an intelligent medical system, the application method being an application of the intelligent medical system (1) as claimed in claim 1, comprising the following steps for diagnosis and treatment:
step c1: an emergency department subsystem (3) notifying a patient to go to a physician's consulting room for treatment;
step c2: the emergency department subsystem (3) reading the identification code of the patient's integrated device (2) and real-time vital sign data from the integrated device (2);
step c3: the emergency department subsystem (3) reading, via the health information subsystem (4), a medical record data corresponding to the identification code of the integrated device (2);
step c4: the emergency department subsystem (3) presenting and displaying the medical record data, the real-time vital sign data, and a comparison of the conditions before and after the treatment on a physician's display device;
step c5: the emergency department subsystem (3) receiving the physician's decision regarding the patient.

11. An application method of an intelligent medical system, the application method being an application of the intelligent medical system (1) as claimed in claim 1, the intelligent medical system (1) further comprising a payment transaction subsystem (5) which is connected to an emergency department subsystem (3) via a network, the application method comprising the following payment steps:
step d1: the payment transaction subsystem (5) calculating a charge to be paid by a patient and sending a payment notification to the emergency department subsystem (3);
step d2: the emergency department subsystem (3) receiving the payment notification;
step d3: the emergency department subsystem (3) transmitting a payment due of the payment notification to an integrated device (2) of the patient and instructing the full-color LED (26) of the integrated device (2) to flash and/or instructing a buzzer (29) of the integrated device (2) to sound to notify the patient;
step d4: a payment machine (11) reading an identification code of the patient's integrated device (2) and the payment due;
step d5: the payment machine (11) determining that the patient has made the payment at the payment machine (11);
step d6: the payment machine (11) notifying the emergency department subsystem (3) that the patient has made the payment at the payment machine (11);
step d7: the emergency department subsystem (3) notifying the payment transaction subsystem (5) that the patient has made the payment.

12. An application method of an intelligent medical system, the application method being an application of the intelligent medical system (1) as claimed in claim 1, the application method comprising the following steps for receiving medication:
step e1: the health information subsystem (4) instructing a pharmacy to dispense medication based on a patient's prescription and to store the dispensed medication to an intelligent medication dispenser (12);
step e2: the intelligent medication dispenser (12) sends a medication availability notification to an emergency department subsystem (3);
step e3: the emergency department subsystem (3) receiving the medication availability notification;
step e4: the emergency department subsystem (3) notifying the patient by instructing full-color LED (26) of the patient's integrated device (2) to flash or/and a buzzer (29) to sound;
step e5: the intelligent medication dispenser (12) reading the identification code of the integrated device (2);
step e6: the intelligent medication dispenser (12) determining whether the patient has received the medication and the instructions for taking the medication;
step e7: The intelligent medication dispenser (12) notifying the emergency department subsystem (3) that the patient has received the medication and instructions for taking the medication.

13. An application method of an intelligent medical system, the application method being an application of the intelligent medical system (1) as claimed in claim 1, the application method comprising the following steps for returning an integrated device (2):
step f1: a return machine (14) reading an identification code of a patient's integrated device (2);
step f2: the return machine (14) sending a return notification and an identification code of the integrated device (2) to an emergency department subsystem (3);
step f3: the emergency department subsystem (3) receiving the return notification and the identification code of the integrated device (2);
step f4: the emergency department subsystem (3) breaking the pairing between the integrated device (2) and the patient's health insurance card.

14. An application method of an intelligent medical system, the application method being an application of the intelligent medical system (1) as claimed in claim 1, the application method comprising the following steps of an intelligent voice flow:
step g1: the emergency department subsystem (3) receiving a voice signal from a patient's integrated device (2);
step g2: an emergency department AI server (7) of the emergency department subsystem (3) performing artificial intelligence speech recognition on the voice signal;
step g3: the emergency department AI server (7) recognizing the patient's request;
step g4: the emergency department subsystem (3) carrying out a follow-up process based on the patient's request.
